# EUROPEAN PATENT APPLICATION

(11) **EP 3 901 600 A1**
(43) Date of publication of application: **27.10.2021**
(21) Application number: 20195884.0
(22) Date of filing: 14.09.2020
(51) Int. Cl.: G01K 13/20, G01K 1/024, A41D 13/11

(54) **BODY TEMPERATURE MONITORING DEVICE, MASK ASSEMBLY, ACCESS CONTROL METHOD, ACCESS CONTROL DEVICE, AND ACCESS CONTROL SYSTEM**

(30) Priority: 24.04.2020 CN 202010335177
(71) Applicant: Beijing Xiaomi Mobile Software Co., Ltd., Beijing 100085 (CN)
(72) Inventor: XIE, Yingchun, Beijing, Beijing 100085 (CN); XING, Zheng, Beijing, Beijing 100085 (CN)
(74) Representative: dompatent von Kreisler Selting Werner - Partnerschaft von Patent- und Rechtsanwälten mbB

(57) **Abstract**

A body temperature monitoring device includes a strap connector and a temperature measurement module mounted to the strap connector. A strap fitting portion of the strap connector is provided with a snap groove fitted with the mask strap. The temperature measurement module is provided on a skin fitting portion of the strap connector.

## Description

### TECHNICAL FIELD

The present invention relates to the field of electronic technology, in particular to a body temperature monitoring device, a mask assembly, an access control method, an access control device, and an access control system.

### BACKGROUND

In the related prior art, smart thermometers, such as infrared thermometers and electronic thermometers, can acquire body temperature data of a user at a certain moment to determine whether a current body temperature of the user is normal. However, the body temperature data of people in different scenes and different moments are dynamically changing and, therefore, the determination on the body temperature status at a certain moment may not be accurate, which degrades the monitoring effect and limits application scenarios of the body temperature data.

In times of influenza virus, new coronavirus and other virus epidemics that can cause abnormal body temperature, temperature measurement is conducted manually through infrared thermometers, electronic thermometers and etc. at an access control station, which not only results in a problem of low accuracy of the body temperature data, but also increases the workload and infection risk of measurers.

### SUMMARY

The present invention provides a body temperature monitoring device, a mask assembly, an access control method, an access control device, and an access control system, to improve an effect of monitoring body temperature data and broaden an application range of the body temperature monitoring device.

The present invention is defined in the independent claims, and the preferable features according to the present invention are defined in the dependent claims. Any embodiment in the present invention that does not fall within the scope of the present invention should be regarded as an example for understanding the present invention.

A first aspect of the present invention provides a body temperature monitoring device configured to work in cooperation with a mask. The mask includes a mask body and a mask strap provided with the mask body. The body temperature monitoring device includes a strap connector including a skin fitting portion and a strap fitting portion provided with at least one snap groove adapted to be fitted with the mask strap; and a temperature measurement module mounted to the strap connector and arranged on the skin fitting portion.

Optionally, the body temperature monitoring device further includes a shaping member fitted with at least a part of the strap connector.

Optionally, the shaping member includes a metal wire arranged within the strap connector.

Optionally, the strap fitting portion is provided with a plurality of snap grooves, the strap connector includes a central section, and the plurality of snap grooves are symmetrically arranged with respect to the central section in a length direction of the strap connector.

Optionally, the temperature measurement module includes a heat conductive element and a temperature sensor, the heat conductive element is arranged on a surface of the skin fitting portion, and the temperature sensor cooperates with the heat conductive element.

Optionally, the strap connector comprises two skin fitting portions located at both ends of the strap connector, each of the two skin fitting portions includes an inner surface and an outer surface arranged oppositely, and the heat conductive element is arranged on one of the inner surface or the outer surface of at least one skin fitting portion of the two skin fitting portions.

Optionally, the heat conductive element includes a metal plate.

Optionally, the temperature measurement module includes an infrared transceiver.

Optionally, the body temperature monitoring device further includes a wireless association module electrically connected to the temperature measurement module and being associated with a mobile apparatus.

A second aspect of the present invention provides a mask assembly. The mask assembly includes a mask and the body temperature monitoring device according to the first aspect of the present invention. The mask includes a mask body and a mask strap provided to the mask body, and the body temperature monitoring device cooperates with the mask strap.

A third aspect of the present invention provides an access control method applied to the body temperature monitoring device according to the first aspect of the present invention or the mask assembly according to the second aspect of the present invention. The access control method includes: acquiring historical body temperature data for a period during which a user wears the body temperature monitoring device or the mask assembly; and sending the historical body temperature data to a mobile apparatus and/or a cloud server. In the context of the present invention the term body temperature data is defined as a temperature of the body of a human, the term historical body temperature data is defined as a plurality of temperatures of the body of a human taken at specific points in time.

A fourth aspect of the present invention proposes an access control device applied to the body temperature monitoring device according to the first aspect of the present invention or the mask assembly according to the second aspect of the present invention. The access control device includes: a first acquiring unit configured to acquire historical body temperature data for a period during which a user wears the body temperature monitoring device or the mask assembly; and a sending unit configured to send the historical body temperature data to a mobile apparatus and/or a cloud server.

A fifth aspect of the present invention proposes an access control system. The access control system includes the body temperature monitoring device according to the first aspect of the present invention or the mask assembly according to the second aspect of the present invention, an access control apparatus, and a cloud server. The body temperature monitoring device or the mask assembly is configured to acquire historical body temperature data during a period during which a user wears the body temperature monitoring device or the mask assembly. The cloud server is configured to store and manage at least a part of preset verification parameters, the preset verification parameters comprise identity information and historical body temperature data corresponding to the identity information, and the part of the preset verification parameters includes the historical body temperature data acquired by the body temperature detection device or the mask assembly and sent to the cloud server. The access control apparatus is coupled to the cloud server and configured to release the access control when receiving an access control releasing signal.

Optionally, the access control apparatus is configured to send the access control releasing signal when the preset verification parameters meet the access control releasing condition.

Optionally, the cloud server is configured to send the access control releasing signal when the preset verification parameters meet the access control releasing condition.

The technical solutions of the present invention have the following beneficial effects.

The body temperature monitoring device of the present invention includes the strap connector and the temperature measurement module mounted to the strap connector. The strap fitting portion of the strap connector is provided with the snap groove adapted to be fitted with the mask strap. The temperature measurement module is provided on the skin fitting portion of the strap connector. During the period when the user wears the mask, the body temperature monitoring device can obtain the user's dynamic historical body temperature data through the temperature measurement module, which improves an effect of monitoring the body temperature data. Since the historical body temperature data obtained by the body temperature monitoring device includes historical body temperature data for the entire period during which the user wears the mask, a body temperature status of the user can be accurately learned through monitoring and analysis of the above historical body temperature data. The accurate body temperature status is used for access control. Hence, the access control has an extra temperature-related verification function, improving the efficiency of access control and enriching the application scenarios of the body temperature monitoring device.

It should be understood that the above general description and the following detailed description are only exemplary and explanatory and cannot be constructed to limit the present invention.

### BRIEF DESCRIPTION OF THE DRAWINGS

The accompanying drawings, which are incorporated in and constitute a part of this specification, illustrate embodiments consistent with the present invention and, together with the description, serve to explain the principles of the present invention.
FIG. 1 is a schematic diagram of a body temperature monitoring device according to an exemplary embodiment.
FIG. 2 is a schematic diagram of a body temperature monitoring device in an unbent state according to an exemplary embodiment.
FIG. 3 is a schematic diagram of a body temperature monitoring device in a bent state according to an exemplary embodiment.
FIG. 4 is a schematic diagram of an exploded view of a body temperature monitoring device according to an exemplary embodiment.
FIG. 5 is a schematic diagram of an application scenario of a body temperature monitoring device according to an exemplary embodiment.
FIG. 6 is a schematic diagram of a mask assembly according to an exemplary embodiment.
FIG. 7 is a flow chart of an access control method according to an exemplary embodiment.
FIG. 8 is a block diagram of an access control device according to an exemplary embodiment.
FIG. 9 is a block diagram of a device for access control according to an exemplary embodiment.
FIG. 10 is a flow chart of an access control method according to another exemplary embodiment.
FIG. 11 is a block diagram of an access control device according to another exemplary embodiment.
FIG. 12 is a block diagram of a device for access control according to another exemplary embodiment.
FIG. 13 is a block diagram of a device for access control according to still another exemplary embodiment.
FIG. 14 is a schematic diagram of an access control method according to an exemplary embodiment.
FIG. 15 is a schematic diagram of an access control method according to another exemplary embodiment.

### DETAILED DESCRIPTION

Exemplary embodiments will be described in detail herein, and examples thereof will be illustrated in accompanying drawings. When the following description refers to the drawings, unless specified otherwise, the same numbers in different drawings represent the same or similar elements. Implementations described in the following exemplary embodiments do not represent all the implementations consistent with the present invention. Instead, they are only examples of devices and methods consistent with some aspects of the present invention recited in the appended claims.

The present invention provides a body temperature monitoring device. The body temperature monitoring device may be used in cooperation with a mask. The mask includes a mask body and mask straps provided with the mask body. The body temperature monitoring device may include: a strap connector and a temperature measurement module mounted to the strap connector. The strap connector may include a skin fitting portion and a strap fitting portion. The temperature measurement module may be arranged at the skin fitting portion. The strap fitting portion may be provided with at least one snap groove that is adapted to be fitted with the mask strap.

For example, the body temperature monitoring device includes the strap connector and the temperature measurement module mounted to the strap connector. The strap fitting portion of the strap connector is provided with the snap groove that is adapted to be fitted with the mask strap. The temperature measurement module is arranged at the skin fitting portion of the strap connector. A user may wear a mask for 4-8 hours in a day, and during the period when the user wears the mask, the body temperature monitoring device can obtain the user's dynamic historical body temperature data through the temperature measurement module, which improves an effect of monitoring the body temperature data. Since the historical body temperature data obtained by the body temperature monitoring device includes historical body temperature data for the entire period during which the user wears the mask, a recent body temperature status of the user can be accurately obtained through monitoring and analysis of the historical body temperature data. The user's recent body temperature status obtained by the body temperature monitoring device may be provided to computers, mobile apparatus, access control apparatus, and cloud servers for further processing, which broadens the application scenarios of the body temperature monitoring device.

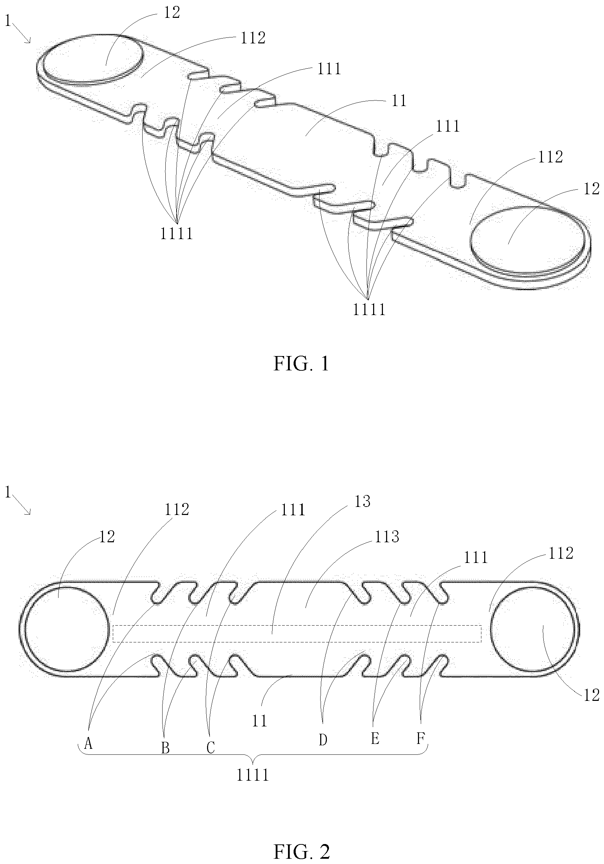

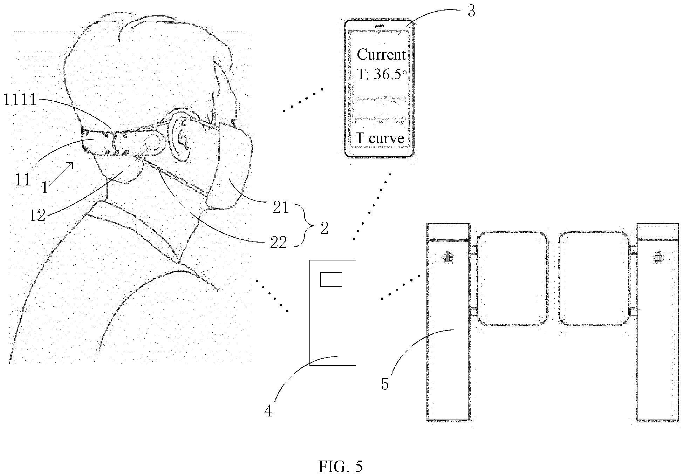

FIG. 1 is a schematic diagram of a body temperature monitoring device 1 according to an exemplary embodiment. As shown in FIG. 1, the body temperature monitoring device 1 includes a strap connector 11 and a temperature measurement module 12 mounted on the strap connector 11. The strap connector 11 includes a skin fitting portion 112 and a strap fitting portion 111. The temperature measurement module 12 is arranged on the skin fitting portion 112. The strap fitting portion 111 is provided with a plurality of snap grooves 1111 adapted to be fitted with a mask strap 22 (Fig. 5).

In some embodiments, shown in Fig. 5, the mask 2 includes a mask body 21 and two mask straps 22 provided on both sides of the mask body 21. The two mask straps 22 are caught in the snap grooves 1111 of the strap connector 11 separately, to realize fixation of the mask body 21. Through the fitting between the snap grooves 1111 of the strap connector 11 and the mask straps 22, the fatigue and pain that may be caused by the mask straps 22 hanging on the user's ears is avoided, and the wearing experience of the mask 2 is improved.

In an embodiment, shown in Fig. 2, the strap connector 11 includes a central section 113, and the plurality of snap grooves 1111 are symmetrically arranged with respect to the central section 113 in a length direction of the strap connector 11. For example, both sides of the central section 113 are individually provided with strap fitting portions 111, and the snap grooves 1111 provided on the strap fitting portion 111 at one side and the snap grooves 1111 provided on the strap fitting portion 111 at the other side are symmetrical in terms of their positions. An extension direction of the strap connector 11 from the central section 113 to the strap fitting portion 111 can be regarded as the length direction of the strap connector 11. The strap connector 11 may have a strip-shaped sheet structure to facilitate deformation, bending and use with the mask straps 22 (Fig. 5).

For example, as shown in FIG. 2, the strap fitting portion 111 provided on one side of the central section 113 has three groups (group A, group B, and group C) of snap grooves 111, and the strap fitting portion 111 provided on the other side of the central section 113 has three groups (group D, group E, group F) of snap grooves 1111 that are symmetrical to the above three groups (group A, group B, and group C) of snap grooves 1111. The groups C, D of snap grooves 1111 are arranged symmetrically with respect to the central section 113; the groups B, E of snap grooves 1111 are arranged symmetrically with respect to the central section 113; and the groups A, F of snap grooves 1111 are arranged symmetrically with respect to the central section 113. The groups A, B, C and the groups D, E, F of snap grooves 1111 are arranged at intervals along the length direction of the strap connector 11. When the mask straps 22 (Fig. 5) are caught in the snap grooves 1111 of different groups, the mask can adapt to different head circumferences to improve wearing comfort. For example, when the two mask straps 22 are hung in the snap grooves 1111 of the groups A and F respectively, the mask can adapt to a head circumference of a first size. When the two mask straps 22 are hung in the snap grooves 1111 of the groups B and E respectively, the mask can adapt to a head circumference of a second size. When the two mask straps 22 are hung in the snap grooves 1111 of the groups C and D respectively, the mask can adapt to a head circumference of a third size. Among them, the first size is larger than the second size, and the second size is larger than the third size. Further, each group may include two snap grooves 1111, and the two snap grooves 1111 are provided on two opposite edges of the strap fitting portion 111, to facilitate the hanging and fitting between the mask strap 22 and the opposite edges of the strap fitting part 111.

Further, the snap groove 1111 may be arranged obliquely with respect to the length direction of the strap connector 11 to form a tree branch-shaped snap groove, so that an extension direction of the snap groove 1111 from a groove bottom to a groove opening is at an obtuse angle with a direction of a pulling force exerted on the mask strap 22, which facilitates the hooking of the mask strap 22 and prevents the mask strap 22 from coming out of the fitting with the snap groove 1111.

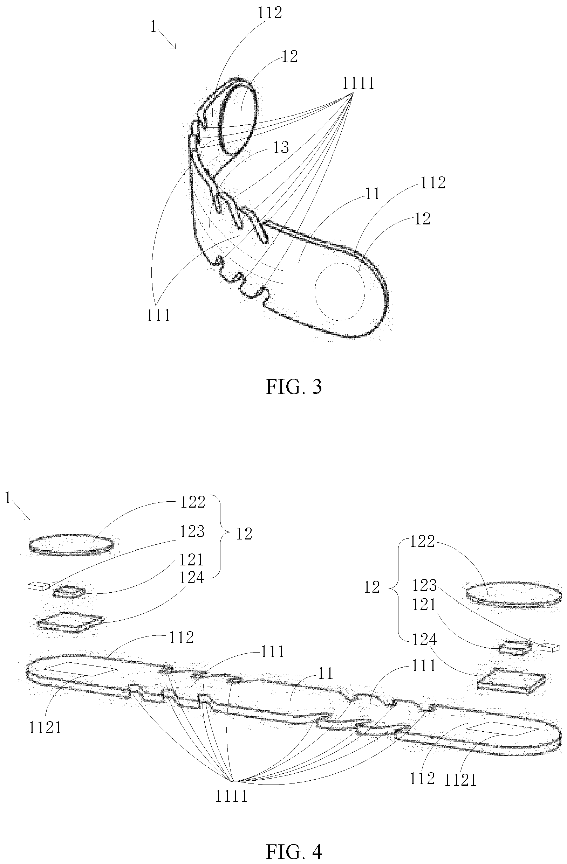

In some embodiments, as shown in FIG. 2 and FIG. 3, the body temperature monitoring device 1 further includes a shaping member 13, and the shaping member 13 is fitted with at least a part of the strap connector 11. The shaping member 13 itself is deformable and can maintain its deformation to cause deformation of the strap connector 11 the same as that of the shaping member 13. For example, the strap connector 11 includes an inner surface and an outer surface that are arranged oppositely, and when the shaping member 13 is bent toward the inner surface of the strap connector 11, it can drive the strap connector 11 to bend toward the inner surface and maintain the current bent state.

The body temperature monitoring device 1 may include one or more shaping members 13. When the body temperature monitoring device 1 includes one shaping member 13, the shaping member 13 can be fitted with the entire section of the strap connector 11 in the length direction, to improve the flexibility of deformation of the strap connector 11. Alternatively, when the body temperature monitoring device 1 includes one or more shaping members 13, the shaping members 13 can also be fitted with the central section 113 and/or two end sections of the strap connector 11, to reduce the cost of the shaping members 13.

In an embodiment, the shaping member 13 may be a metal wire arranged within the strap connector 11, the characteristics of the metal wire -deformability and maintaining the deformation - are used to make the strap connector 11 to deform to adapt to the user. The metal wire has good bendability and high structural strength. In addition, the material of the strap connector 11 can be plastic, soft rubber or the like, to improve the touch sensation between the strap connector and the skin and improve the wearing experience of the body temperature monitoring device 1.

FIG. 4 is a schematic diagram of an exploded view of the body temperature monitoring device 1 according to an exemplary embodiment. FIG. 5 is a schematic diagram of an application scenario of the body temperature monitoring device 1 according to an exemplary embodiment. In FIG. 5, dotted lines among the body temperature monitoring device 1, a mobile apparatus 3, a cloud server 4, and an access control apparatus 5 represent association relationships among them, which can realize data transmission. As shown in FIGS. 4, the temperature measurement module 12 includes a heat conductive element 122 and a temperature sensor 121, the heat conductive element 122 is disposed on a surface of the skin fitting portion 112, and the temperature sensor 121 cooperates with the heat conductive element 122. That is, the heat conductive element 122 is in contact with the skin to perform heat conduction, and the temperature sensor 121 obtains the heat from the heat conductive element 122 to acquire the body temperature data.

The skin fitting portions 112 may be located at both ends of the strap connector 11. The skin fitting portion 112 includes an inner surface and an outer surface arranged oppositely. During the use of the body temperature monitoring device 1, one of the inner surface and the outer surface of the skin fitting portion 112 faces the skin and the other thereof faces the outside. The heat conductive element 122 may be provided on the inner surface and/or the outer surface of at least one skin fitting portion 112. For example, in an embodiment, the temperature measurement module 12 includes two heat conductive elements 122 and two temperature sensors 121, the two heat conductive elements 122 are provided on respective inner surfaces of the two skin fitting portions 112, and the two temperature sensors 121 are arranged under the heat conductive elements 122 correspondingly to facilitate the acquisition of the temperatures of the corresponding heat conductive element 122. When the user wears the mask 2 and the body temperature monitoring device 1, the skin fitting portions 112 at both ends of the strap connector 11 correspond to the skin behind the user's ears, and the heat conductive elements 122 provided on the skin fitting portions 112 can be in touch with the skin behind the ears to perform heat conduction. The configuration of the two heat conductive elements 122 adds collection of body temperature data, which is beneficial to improving the accuracy of body temperature monitoring.

In another embodiment, the temperature measurement module 12 may include one heat conductive element 122 and one temperature sensor 121. The one heat conductive element 122 is provided on an inner surface of one skin fitting portion 112, and the temperature sensor 121 is arranged under the heat conductive element 122 to obtain the temperature of the heat conductive element 122. When the user wears the mask 2 and the body temperature monitoring device 1, the skin fitting portion 112 at one end of the strap connector 11 corresponds to the skin behind the user's ears, and the heat conductive element 122 provided on the skin fitting portion 112 can be in touch with the skin behind the ears to perform heat conduction. The configuration of one heat conductive element 122 and one temperature sensor 121 reduces the cost of the body temperature monitoring device 1 on the basis of ensuring the accuracy of body temperature monitoring.

In an embodiment, the heat conductive element 122 may be a metal plate, and the metal plate may be round or square, or have other irregularly shapes, which is not limited in the present invention. The metal plate can be mounted to the strap connector 11 by means of clamping, bonding, or the like. The metal plate may be a steel plate or a plate-like structure of other metals, which is not limited in the present invention. Alternatively, in other embodiments, the heat conductive element 122 may also be made of other materials capable of performing heat conduction.

In some other embodiments, the temperature measurement module 12 may be an infrared transceiver to receive infrared radiation from the human body and convert the received energy of infrared radiation into an electrical signal. The energy of infrared radiation is related to the human body temperature, so the electrical signal converted from the infrared radiation energy can reflect the human body temperature.

In some embodiments, the skin fitting portion 112 of the strap connector 11 can be provided with a receiving groove 1121, and at least a part of the temperature measurement module 12 is received in the receiving groove 1121. When the temperature measurement module 12 includes the heat conductive element 122 and the temperature sensor 121, the temperature sensor 121 is received in the receiving groove 1121, and a part of the heat conductive element 122 may protrude from the receiving groove 1121 to facilitate contact with the skin to achieve heat conduction. When the temperature measurement module 12 is an infrared transceiver, the infrared transceiver can also be mounted in the receiving groove 1121.

In some embodiments, as shown in FIGS. 4 and 5, the body temperature monitoring device 1 may further include a wireless association module 123. The wireless association module 123 is electrically connected to the temperature measurement module 12, and the wireless association module 123 is associated with the mobile apparatus 3 to send the historical body temperature data collected by the temperature measurement module 12 to the mobile apparatus 3 or the cloud server 4. The mobile apparatus 3 or the cloud server 4 can perform temperature-related control over the access control apparatus 5 and other travel equipment by using the stored historical body temperature data. Alternatively, the body temperature monitoring device 1 can also receive a control instruction from an external device such as the mobile apparatus 3 through the temperature measurement module 12. The wireless association module 123 may be arranged in the receiving groove 1121 to facilitate electrical connection with the temperature measurement module 12.

In some embodiments, the wireless association module 123 may be at least one of a Bluetooth module, a wireless fidelity (Wi-Fi) module, and a ZigBee module. The mobile apparatus 3 may be a mobile phone, a tablet computer, a vehicle-mounted terminal, a medical terminal or the like, which is not limited in the present invention. For example, when the mobile apparatus 3 is a mobile phone, the user's body temperature data obtained by the body temperature monitoring device 1 can be synchronized to the mobile phone in real time, so that the user can check the real-time body temperature data and historical body temperature data by software on the mobile phone.

The body temperature monitoring device 1 may also include a power supply module 124. The power supply module 124 may be electrically connected to the wireless association module 123 and the temperature measurement module 12 to provide durable electric energy for the wireless association module 123 and the temperature measurement module 12. The power supply module 124 may also be arranged in the receiving groove 1121 to facilitate electrical connection with the wireless association module 123 and the temperature measurement module 12. The temperature measurement module 12 and the wireless association module 123 can be packaged into an integrated structure and arranged above the power supply module 124 to reduce space occupation and facilitate the electrical connection between the power supply module 124 and the wireless association module 123, as well as the temperature measurement module 12.

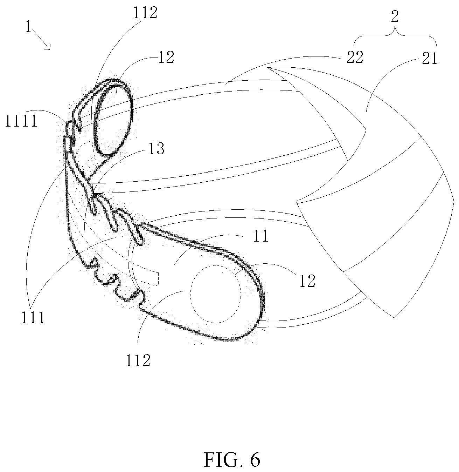

The present invention also provides a mask assembly. FIG. 6 is a schematic diagram of a mask assembly according to an exemplary embodiment. As shown in FIG. 6, the mask assembly includes a mask 2 and a body temperature monitoring device 1. The mask 2 includes a mask body 21 and a mask strap 22 provided with the mask body 21. The body temperature monitoring device 1 cooperates with the mask strap 22.

The body temperature monitoring device 1 includes a strap connector 11 and a temperature measurement module 12 mounted to the strap connector 11. A strap fitting portion 111 of the strap connector 11 is provided with a snap groove 1111 fitted with the mask strap 22. The temperature measurement module 12 is provided on a skin fitting portion 112 of the strap connector 11. During the period when the user wears the mask 2, the body temperature monitoring device 1 can obtain the user's dynamic historical body temperature data through the temperature measurement module 12, which improves an effect of monitoring the body temperature data. Since the historical body temperature data obtained by the body temperature monitoring device 1 includes historical body temperature data for the entire period during which the user wears the mask, a body temperature status of the user can be accurately learned through monitoring and analysis of the above historical body temperature data, and the application scenarios of the body temperature monitoring device and the body temperature data obtained by it are broadened.

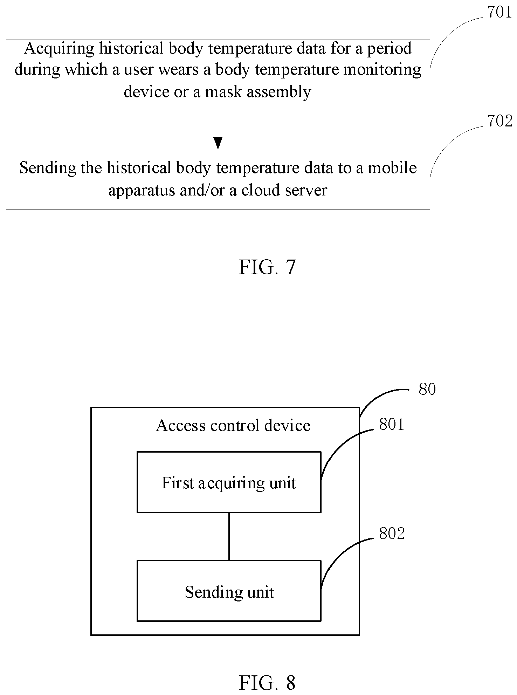

The present invention also provides an access control method, in which the body temperature monitoring device or the mask assembly described above is used. FIG. 7 is a flow chart of an access control method according to an exemplary embodiment. As shown in FIG. 7, the access control method may include the following operations.

In block 701, historical body temperature data for a period during which the user wears the body temperature monitoring device or the mask assembly is acquired.

In block 702, the historical body temperature data is sent to a mobile apparatus and/or a cloud server.

In the above embodiment, the body temperature monitoring device may be associated with the mobile apparatus or the cloud server through the wireless association module, so as to send the acquired historical body temperature data during the period when the user wears the body temperature monitoring device or the mask assembly to the mobile apparatus and/or the cloud server.

Since the historical body temperature data acquired by the body temperature monitoring device is dynamic data for a period, when the historical body temperature data is sent to the mobile apparatus, the user's body temperature change can be viewed on the mobile apparatus, and the body temperature status can be monitored more accurately and intuitively. When the historical body temperature data is sent to the cloud server, the user's historical body temperature data can be managed and stored by the cloud server, which is helpful for the application of the historical body temperature data on other equipment such as the access control apparatus.

Corresponding to the foregoing embodiment of the access control method, the present invention also provides an access control device. The access control device is applied to the above body temperature monitoring device or the mask assembly. FIG. 8 is a block diagram of an access control device 80 according to an exemplary embodiment. As shown in FIG. 8, the access control device 80 can include a first acquiring unit 801 and a sending unit 802.

The first acquiring unit 801 is configured to acquire historical body temperature data for a period during which the user wears the body temperature monitoring device or the mask assembly.

The sending unit 802 is configured to send the historical body temperature data to the mobile apparatus and/or a cloud server.

In the above embodiment, the body temperature monitoring device can be associated with the mobile apparatus or the cloud server through the wireless association module, so as to send the acquired historical body temperature data during the period when the user wears the body temperature monitoring device or the mask assembly to the mobile apparatus and/or the cloud server.

Since the historical body temperature data acquired by the body temperature monitoring device is dynamic data for a period, when the historical body temperature data is sent to the mobile apparatus, the user's body temperature change can be viewed on the mobile apparatus, and the body temperature status can be monitored more accurately and intuitively. When the historical body temperature data is sent to the cloud server, the user's historical body temperature data can be managed and stored by the cloud server, which is helpful for the application of the historical body temperature data on other equipment such as the access control apparatus.

Regarding the device in the foregoing embodiment, the specific manner in which each unit performs operations has been described in detail in the embodiment regarding the method, and hence will not be repeated.

The units described as separate components may or may not be physically separate, and the components illustrated as units may or may not be physical units, that is, they may be located in one place or may be distributed in multiple networks. Some or all of the units can be selected according to actual needs.

The present invention also provides an access control device, including: a processor; and a memory for storing instructions executable by the processor. The processor is configured to acquire historical body temperature data for a period during which the user wears the body temperature monitoring device or the mask assembly; and send the historical body temperature data to the mobile apparatus and/or a cloud server.

The present invention also provides a terminal. The terminal includes a processor; and a memory for storing one or more programs to be executed by the processor. The one or more programs include instructions for performing the following operations, acquiring historical body temperature data for a period during which the user wears the body temperature monitoring device or the mask assembly, and sending the historical body temperature data to mobile apparatus and/or a cloud server.

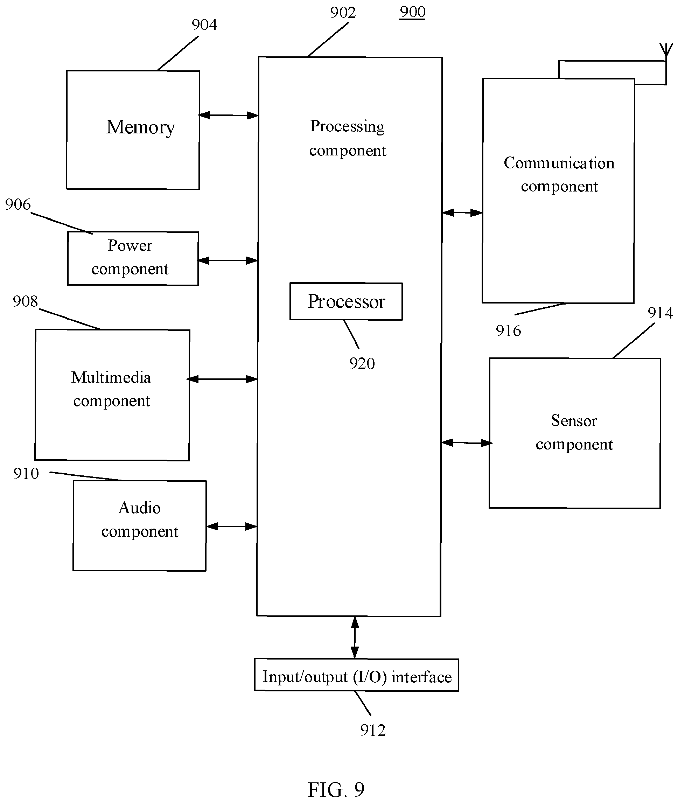

FIG. 9 is a block diagram of a device 900 for access control according to an exemplary embodiment. For example, the device 900 may be a mobile phone, a computer, a digital broadcast terminal, a messaging device, a gaming console, a tablet, a medical device, exercise equipment, a personal digital assistant, and the like.

Referring to FIG. 9, the device 900 may include one or more of the following components: a processing component 902, a memory 904, a power component 906, a multimedia component 908, an audio component 910, an input/output (I/O) interface 912, a sensor component 914, and a communication component 916.

The processing component 902 typically controls overall operations of the device 900, such as the operations associated with display, telephone calls, data communications, camera operations, and recording operations. The processing component 902 may include one or more processors 920 to execute instructions to perform all or part of the steps in the above described methods. Moreover, the processing component 902 may include one or more modules which facilitate the interaction between the processing component 902 and other components. For instance, the processing component 902 may include a multimedia module to facilitate the interaction between the multimedia component 908 and the processing component 902.

The memory 904 is configured to store various types of data to support the operation of the device 900. Examples of such data include instructions for any applications or methods operated on the device 900, contact data, phonebook data, messages, pictures, video, etc. The memory 904 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 906 provides power to various components of the device 900. The power component 906 may include a power management system, one or more power sources, and any other components associated with the generation, management, and distribution of power in the device 900.

The multimedia component 908 includes a screen providing an output interface between the device 900 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 908 includes a front camera and/or a rear camera. The front camera and the rear camera may receive an external multimedia datum while the device 900 is in an operation mode, such as a photographing mode or a video mode. Each of the front camera and the rear camera may be a fixed optical lens system or have focus and optical zoom capability.

The audio component 910 is configured to output and/or input audio signals. For example, the audio component 910 includes a microphone ("MIC") configured to receive an external audio signal when the device 900 is in an operation mode, such as a call mode, a recording mode, and a voice recognition mode. The received audio signal may be further stored in the memory 904 or transmitted via the communication component 916. In some embodiments, the audio component 910 further includes a speaker to output audio signals.

The I/O interface 912 provides an interface between the processing component 902 and peripheral interface modules, such as a keyboard, a click wheel, buttons, and the like. The buttons may include, but are not limited to, a home button, a volume button, a starting button, and a locking button.

The sensor component 914 includes one or more sensors to provide status assessments of various aspects of the device 900. For instance, the sensor component 914 may detect an open/closed status of the device 900, relative positioning of components, e.g., the display and the keypad, of the device 900, a change in position of the device 900 or a component of the device 900, a presence or absence of user contact with the device 900, an orientation or an acceleration/deceleration of the device 900, and a change in temperature of the device 900. The sensor component 914 may include a proximity sensor configured to detect the presence of nearby objects without any physical contact. The sensor component 914 may also include a light sensor, such as a CMOS or CCD image sensor, for use in imaging applications. In some embodiments, the sensor component 914 may also include an accelerometer sensor, a gyroscope sensor, a magnetic sensor, a pressure sensor, or a temperature sensor.

The communication component 916 is configured to facilitate communication, wired or wirelessly, between the device 900 and other devices. The device 900 can access a wireless network based on a communication standard, such as Wi-Fi, 2G, 3G, 4G LTE, 5G NR or a combination thereof. In one exemplary embodiment, the communication component 916 receives a broadcast signal or broadcast associated information from an external broadcast management system via a broadcast channel. In one exemplary embodiment, the communication component 916 further includes a near field communication (NFC) module to facilitate short-range communications. In one exemplary embodiment, the communication component 916 may be implemented based on a radio frequency identification (RFID) technology, an infrared data association (IrDA) technology, an ultra-wideband (UWB) technology, a Bluetooth (BT) technology, and other technologies.

In exemplary embodiments, the device 900 may be implemented with one or more application specific integrated circuits (ASICs), digital signal processors (DSPs), digital signal processing devices (DSPDs), programmable logic devices (PLDs), field programmable gate arrays

(FPGAs), controllers, micro-controllers, microprocessors, or other electronic components, for performing the above described methods.

In exemplary embodiments, there is also provided a non-transitory computer-readable storage medium including instructions, such as included in the memory 904, executable by the processor 920 in the device 900, for performing the above-described methods. For example, the non-transitory computer-readable storage medium may be a read only memory (ROM), a random access memory (RAM), a compact disc read - only memory (CD-ROM), a magnetic tape, a floppy disc, an optical data storage device, and the like.

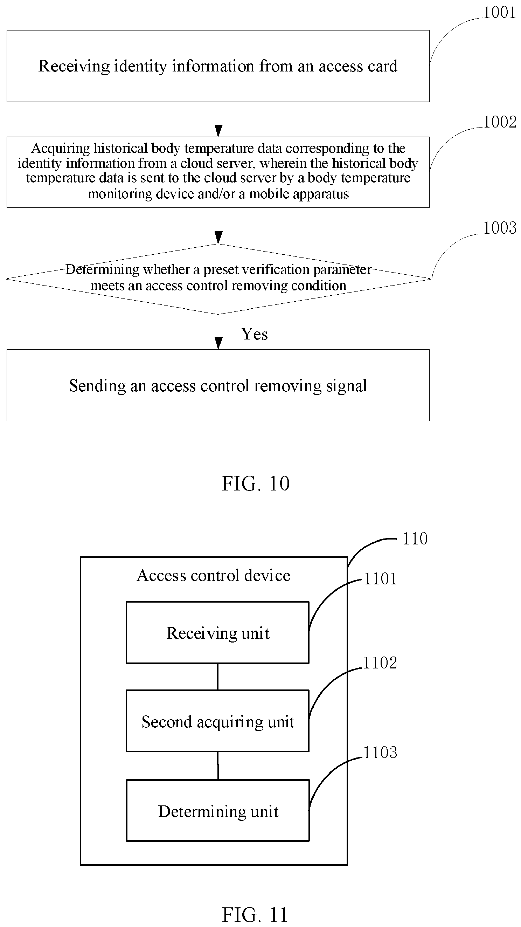

The present invention also provides an access control method that is applied to access control apparatus. FIG. 10 is a flow chart of an access control method according to an exemplary embodiment. As shown in FIG. 10, the access control method can include the following actions.

In block 1001, identity information from an access card is received.

The access card contains the user's identity information. When the access card is swiped on the access control apparatus, the access control apparatus can receive the identity information in the access card. The access card may be an access card of a community or an office building, or a company's electronic work card.

In block 1002, historical body temperature data corresponding to the identity information is acquired from a cloud server, in which the historical body temperature data is sent to the cloud server by a body temperature monitoring device and/or the mobile apparatus.

In block 1003, it is determined whether a preset verification parameter meets an access control releasing condition, and if so, an access control releasing signal is sent (1004) to permit the user's access to, e.g., a place. The preset verification parameter at least includes the identity information and the historical body temperature data corresponding to the identity information.

The corresponding historical body temperature data is acquired from the cloud server according to the identity information provided by the access card, and the historical body temperature data is sent to the cloud server by the body temperature monitoring device and/or the mobile apparatus. That is, the binding and recognition of the user's historical body temperature data is realized through the access card, and the identity information and the historical body temperature data corresponding to the identity information are used as one of the preset verification parameters of the access control apparatus. The access control apparatus is additionally equipped with a body temperature verification function, which improves the functional diversity and work efficiency of the access control apparatus.

Corresponding to the foregoing embodiment of the access control method, the present invention also provides an access control device. The access control device is applied to the access control apparatus. FIG. 11 is a block diagram of an access control device 110 according to an exemplary embodiment. As shown in FIG. 11, the access control device 110 includes a receiving unit 1101, a second acquiring unit 1102, and a determining unit 1103.

The receiving unit 1101 is configured to receive identity information from an access card.

The second acquiring unit 1102 is configured to acquire historical body temperature data corresponding to the identity information from a cloud server, in which the historical body temperature data is sent to the cloud server by the body temperature monitoring device and/or the mobile apparatus.

The determining unit 1103 is configured to determine whether a preset verification parameter meets an access control releasing condition, and if so, an access control releasing signal is sent, in which the preset verification parameter at least includes the identity information and the historical body temperature data corresponding to the identity information.

The corresponding historical body temperature data is acquired from the cloud server according to the identity information provided by the access card, and the historical body temperature data is sent to the cloud server by the body temperature monitoring device and/or the mobile apparatus. That is, the binding and recognition of the user's historical body temperature data is realized through the access card, and the identity information and the historical body temperature data corresponding to the identity information are used as one of the preset verification parameters of the access control apparatus. The access control apparatus is additionally equipped with a body temperature verification function, which improves the functional diversity and work efficiency of the access control apparatus.

Regarding the device in the foregoing embodiment, the specific manner in which each unit performs operations has been described in detail in the embodiment regarding the method, and hence will not be repeated.

The units described as separate components may or may not be physically separate, and the components illustrated as units may or may not be physical units, that is, they may be located in one place or may be distributed in multiple networks. Some or all of the units can be selected according to actual needs.

The present invention also provides an access control device, including: a processor; and a memory for storing instructions executable by the processor. The processor is configured to receive identity information from an access card; acquire historical body temperature data corresponding to the identity information from a cloud server, in which the historical body temperature data is sent to the cloud server by the body temperature monitoring device and/or the mobile apparatus; and determine whether a preset verification parameter meets an access control releasing condition, and if so, send an access control releasing signal. The preset verification parameter at least includes the identity information and the historical body temperature data corresponding to the identity information.

The present invention also provides a terminal. The terminal includes a processor; and a memory for storing one or more programs to be executed by the processor. The one or more programs include instructions for performing the following operations, receiving identity information from an access card; acquiring historical body temperature data corresponding to the identity information from a cloud server, in which the historical body temperature data is sent to the cloud server by the body temperature monitoring device and/or the mobile apparatus; and determining whether a preset verification parameter meets an access control releasing condition, and if so, sending an access control releasing signal, in which the preset verification parameter at least includes the identity information and the historical body temperature data corresponding to the identity information.

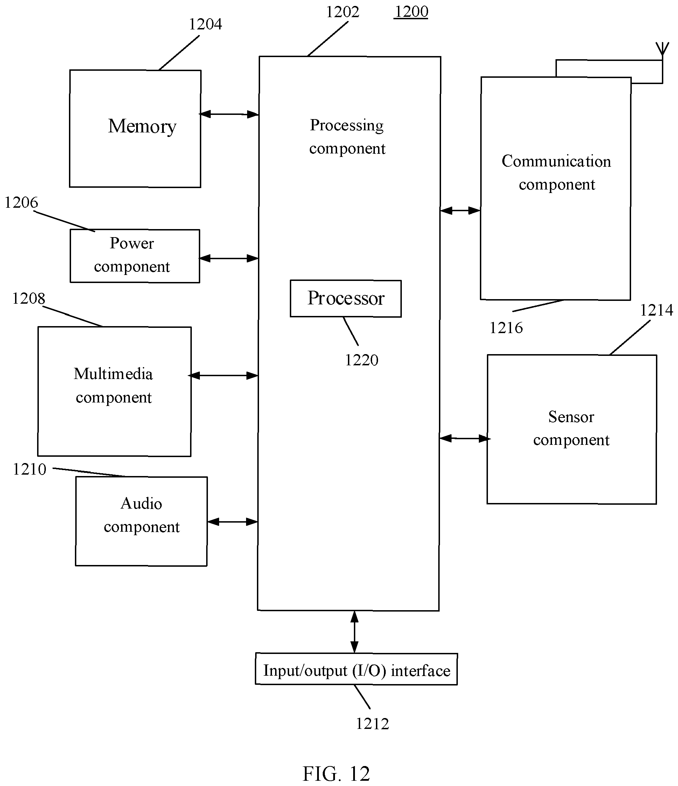

FIG. 12 is a block diagram of a device 1200 for access control according to an exemplary embodiment of the present invention. For example, the device 1200 may be a mobile phone, a computer, a digital broadcast terminal, a messaging device, a gaming console, a tablet, a medical device, exercise equipment, a personal digital assistant, and the like.

Referring to FIG. 12, the device 1200 may include one or more of the following components: a processing component 1202, a memory 1204, a power component 1206, a multimedia component 1208, an audio component 1210, an input/output (I/O) interface 1212, a sensor component 1214, and a communication component 1216.

The processing component 1202 typically controls overall operations of the device 1200, such as the operations associated with display, telephone calls, data communications, camera operations, and recording operations. The processing component 1202 may include one or more processors 1220 to execute instructions to perform all or part of the steps in the above described methods. Moreover, the processing component 1202 may include one or more modules which facilitate the interaction between the processing component 1202 and other components. For instance, the processing component 1202 may include a multimedia module to facilitate the interaction between the multimedia component 1208 and the processing component 1202.

The memory 1204 is configured to store various types of data to support the operation of the device 1200. Examples of such data include instructions for any applications or methods operated on the device 1200, contact data, phonebook data, messages, pictures, video, etc. The memory 1204 may be implemented using any type of volatile or non-volatile memory devices, or a combination thereof, such as a static random access memory (SRAM), an electrically erasable programmable read-only memory (EEPROM), an erasable programmable read-only memory (EPROM), a programmable read-only memory (PROM), a read-only memory (ROM), a magnetic memory, a flash memory, a magnetic or optical disk.

The power component 1206 provides power to various components of the device 1200. The power component 1206 may include a power management system, one or more power sources, and any other components associated with the generation, management, and distribution of power in the device 1200.

The multimedia component 1208 includes a screen providing an output interface between the device 1200 and the user. In some embodiments, the screen may include a liquid crystal display (LCD) and a touch panel (TP). If the screen includes the touch panel, the screen may be implemented as a touch screen to receive input signals from the user. The touch panel includes one or more touch sensors to sense touches, swipes, and gestures on the touch panel. The touch sensors may not only sense a boundary of a touch or swipe action, but also sense a period of time and a pressure associated with the touch or swipe action. In some embodiments, the multimedia component 1208 includes a front camera and/or a rear camera. The front camera and the rear camera may receive an external multimedia datum while the device 1200 is in an operation mode, such as a photographing mode or a video mode. Each of the front camera and the rear camera may be a fixed optical lens system or have focus and optical zoom capability.

The audio component 1210 is configured to output and/or input audio signals. For example, the audio component 1210 includes a microphone ("MIC") configured to receive an external audio signal when the device 1200 is in an operation mode, such as a call mode, a recording mode, and a voice recognition mode. The received audio signal may be further stored in the memory 1204 or transmitted via the communication component 1216. In some embodiments, the audio component 1210 further includes a speaker to output audio signals.

The I/O interface 1212 provides an interface between the processing component 1202 and peripheral interface modules, such as a keyboard, a click wheel, buttons, and the like. The buttons may include, but are not limited to, a home button, a volume button, a starting button, and a locking button.

The sensor component 1214 includes one or more sensors to provide status assessments of various aspects of the device 1200. For instance, the sensor component 1214 may detect an open/closed status of the device 1200, relative positioning of components, e.g., the display and the keypad, of the device 1200, a change in position of the device 1200 or a component of the device 1200, a presence or absence of user contact with the device 1200, an orientation or an acceleration/deceleration of the device 1200, and a change in temperature of the device 1200. The sensor component 1214 may include a proximity sensor configured to detect the presence of nearby objects without any physical contact. The sensor component 1214 may also include a light sensor, such as a CMOS or CCD image sensor, for use in imaging applications. In some embodiments, the sensor component 1214 may also include an accelerometer sensor, a gyroscope sensor, a magnetic sensor, a pressure sensor, or a temperature sensor.

The communication component 1216 is configured to facilitate communication, wired or wirelessly, between the device 1200 and other devices. The device 1200 can access a wireless network based on a communication standard, such as Wi-Fi, 2G, 3G, 4G LTE, 5G NR or a combination thereof. In one exemplary embodiment, the communication component 1216 receives a broadcast signal or broadcast associated information from an external broadcast management system via a broadcast channel. In one exemplary embodiment, the communication component 1216 further includes a near field communication (NFC) module to facilitate short-range communications. For example, the NFC module may be implemented based on a radio frequency identification (RFID) technology, an infrared data association (IrDA) technology, an ultra-wideband (UWB) technology, a Bluetooth (BT) technology, and other technologies.

In exemplary embodiments, the device 1200 may be implemented with one or more application specific integrated circuits (ASICs), digital signal processors (DSPs), digital signal processing devices (DSPDs), programmable logic devices (PLDs), field programmable gate arrays (FPGAs), controllers, micro-controllers, microprocessors, or other electronic components, for performing the above described methods.

In exemplary embodiments, there is also provided a non-transitory computer-readable storage medium including instructions, such as included in the memory 1204, executable by the processor 1220 in the device 1200, for performing the access control method applied to the access control apparatus. For example, the non-transitory computer-readable storage medium may be a ROM, a RAM, a CD-ROM, a magnetic tape, a floppy disc, an optical data storage device, and the like.

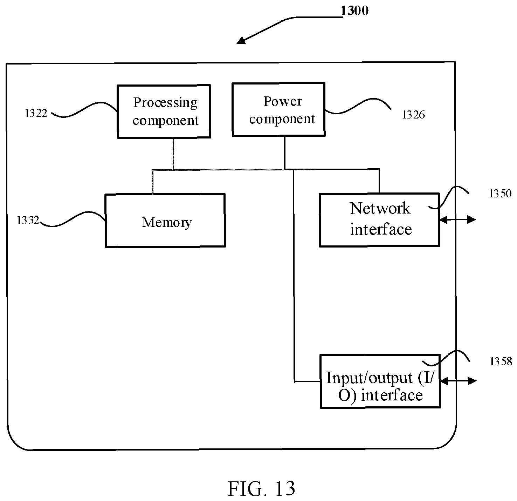

FIG. 13 is a block diagram of a device 1300 for access control according to still another exemplary embodiment of the present invention. For example, the device 1300 may be provided as a server. Referring to FIG. 13, the device 1300 includes a processing component 1322 that further includes one or more processors; and a memory resource represented by a memory 1332, which is configured to store instructions that can be executed by the processing component 1322, such as application programs. The application programs stored in the memory 1332 may include one or more modules each corresponding to a set of instructions. In addition, the processing component 1322 is configured to execute instructions to perform the aforementioned method.

The device 1300 may also include a power component 1326 configured to perform power management for the device 1300, a wired or wireless network interface 1350 configured to couple the device 1300 to a network, and an input/output (I/O) interface 1358. The device 1300 can operate an operating system stored in the memory 1332, such as Windows ServerTM, Mac OS XTM, UnixTM, LinuxTM, FreeBSDTM, or the like.

The present invention also provides an access control system that includes the above body temperature monitoring device, the access control apparatus, and a cloud server. The body temperature monitoring device is configured to acquire historical body temperature data during a period during which the user wears the body temperature monitoring device or the mask assembly. The cloud server is configured to store and manage at least a part of preset verification parameters, and the part of the preset verification parameters includes the historical body temperature data sent to the cloud server by the body temperature monitoring device and/or the mobile apparatus. The access control apparatus is coupled to the cloud server to determine whether the preset verification parameters meet an access control releasing condition, and if so, an access control releasing signal is sent, and the access control is released according to the access control releasing signal. The preset verification parameters at least include the identity information and the historical body temperature data corresponding to the identity information.

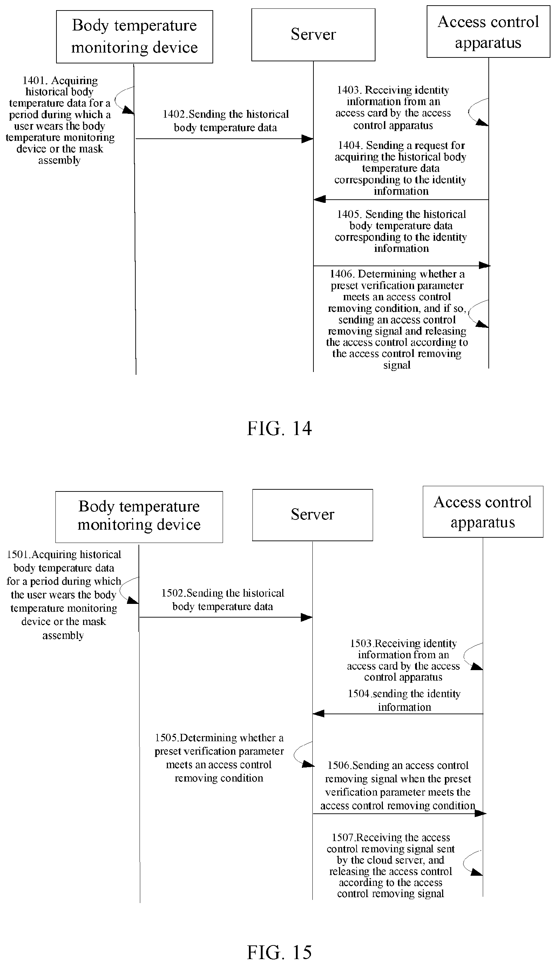

FIG. 14 is a schematic diagram of an access control method according to an exemplary embodiment. The method may include the following operations.

In block 1401, a body temperature monitoring device acquires historical body temperature data for a period during which the user wears the body temperature monitoring device or a mask assembly.

In block 1402, the body temperature monitoring device sends the historical body temperature data to a cloud server.

In block 1403, an access control apparatus receives identity information from an access card, in which the access card contains the user's identity information. When the access card is swiped on the access control apparatus, the access control apparatus can receive the identity information in the access card. The access card may be an access card of a community or an office building, or a company's electronic work card.

In block 1404, the access control apparatus sends a request for acquiring the historical body temperature data corresponding to the identity information to the cloud server.

In block 1405, the cloud server sends the historical body temperature data corresponding to the identity information to the access control apparatus.

In block 1406, the access control apparatus determines whether a preset verification parameter meets an access control releasing condition, and if so, an access control releasing signal is sent, and the access control is released according to the access control releasing signal to permit the user's access to, e.g., a place. The preset verification parameter at least includes the identity information and the historical body temperature data corresponding to the identity information.

In the above embodiment, the body temperature monitoring device can be associated with the mobile apparatus or the cloud server through the wireless association module, so as to send the acquired historical body temperature data during the period when the user wears the body temperature monitoring device or the mask assembly to the cloud server.

Since the historical body temperature data acquired by the body temperature monitoring device is dynamic data for a period, when the historical body temperature data is sent to the cloud server, the user's historical body temperature data can be managed and stored by the cloud server, which is helpful for the application of the historical body temperature data on other equipment such as the access control apparatus.

The present invention also provides an access control system that includes a body temperature monitoring device, an access control apparatus, and a cloud server. The body temperature monitoring device is configured to acquire historical body temperature data during a period during which the user wears the body temperature monitoring device or the mask assembly. The cloud server is configured to store and manage at least a part of preset verification parameters, determine whether the preset verification parameters meet an access control releasing condition, and if so, send an access control releasing signal. The preset verification parameters at least include the identity information and the historical body temperature data corresponding to the identity information. The aforementioned part of the preset verification parameters includes the historical body temperature data sent to the cloud server by the body temperature monitoring device and/or the mobile apparatus. The access control apparatus is coupled to the cloud server and is configured to release the access control when receiving the access control releasing signal.

FIG. 15 is a schematic diagram of an access control method according to another exemplary embodiment. The method may include the following operations.

In block 1501, a body temperature monitoring device acquires historical body temperature data for a period during which the user wears the body temperature monitoring device or the mask assembly.

In block 1502, the body temperature monitoring device sends the historical body temperature data to a cloud server.

In block 1503, an access control apparatus receives identity information from an access card.

The access card contains the user's identity information. When the access card is swiped on the access control apparatus, the access control apparatus can receive the identity information in the access card. The access card may be an access card of a community or an office building, or a company's electronic work card.

In block 1504, the access control apparatus sends the identity information to the cloud server.

In block 1505, the cloud server determines whether a preset verification parameter meets an access control releasing condition. The preset verification parameter at least includes the identity information and the historical body temperature data corresponding to the identity information.

In block 1506, when the preset verification parameter meets the access control releasing condition, the cloud server sends an access control releasing signal to the access control apparatus.

In block 1507, the access control apparatus receives the access control releasing signal sent by the cloud server, and releases the access control according to the access control releasing signal to permit the user's access to, e.g., a place.

In the above embodiment, the body temperature monitoring device can be associated with the mobile apparatus or the cloud server through the wireless association module, so as to send the acquired historical body temperature data during the period when the user wears the body temperature monitoring device or the mask assembly to the cloud server.

Since the historical body temperature data acquired by the body temperature monitoring device is dynamic data for a period, when the historical body temperature data is sent to the cloud server, the user's historical body temperature data can be managed and stored by the cloud server, which is helpful for the application of the historical body temperature data on other equipment such as the access control apparatus. Utilizing the cloud server to determine whether the preset verification parameter meets the access control releasing condition can reduce the information forwarding process, improve the efficiency of judgment on the preset verification parameters, and simplify control action for the access control apparatus.

Embodiments also provide an access control method applied to an access control apparatus. The access control method includes: receiving identity information from an access card; acquiring historical body temperature data corresponding to the identity information from a cloud server, wherein the historical body temperature data is sent to the cloud server by a body temperature monitoring device and/or a mobile apparatus; and determining whether a preset verification parameter meets an access control releasing condition, and sending an access control releasing signal when the preset verification parameter meets the access control releasing condition, wherein the preset verification parameter at least includes the identity information and the historical body temperature data corresponding to the identity information.

Embodiments also provide an access control device applied to an access control apparatus. The access control device includes: a receiving unit configured to receive identity information from an access card; a second acquiring unit configured to acquire historical body temperature data corresponding to the identity information from a cloud server, wherein the historical body temperature data is sent to the cloud server by a body temperature detection device and/or a mobile apparatus; and a determining unit configured to determine whether a preset verification parameter meets an access control releasing condition, and send an access control removing signal when the preset verification parameter meets the access control releasing condition, wherein the preset verification parameter at least includes the identity information and the historical body temperature data corresponding to the identity information.

Embodiments also provide an access control system. The access control system includes the body temperature monitoring device, an access control apparatus, and a cloud server. The body temperature monitoring device is configured to acquire historical body temperature data during a period during which a user wears the body temperature monitoring device or the mask assembly. The cloud server is configured to store and manage at least a part of preset verification parameters, determine whether the preset verification parameters meet an access control releasing condition, and send an access control removing signal when the preset verification parameters meet the access control releasing condition, wherein the preset verification parameters at least include identity information and the historical body temperature data corresponding to the identity information, and the part of the preset verification parameters includes the historical body temperature data sent to the cloud server by the body temperature detection device and/or a mobile apparatus. The access control apparatus is coupled to the cloud server and configured to release the access control when receiving the access control removing signal.

Embodiments also provide a computer readable storage medium stored with a computer instruction. When the computer instruction is executed by a processor, actions of the method applied to the body temperature monitoring device or the mask assembly are implemented.

Embodiments also provide a computer readable storage medium stored with a computer instruction. When the computer instruction is executed by a processor, actions of the method applied to the access control apparatus are implemented.

Other embodiments of the present invention will be apparent to those skilled in the art from consideration of the specification and practice of the present invention. This application is intended to cover any variations, uses, or adaptations of the present invention, which are in accordance with the general principles of the present invention and include common knowledge or conventional technical means in the art that are not disclosed herein. The specification and embodiments are considered to be exemplary only, and the true scope of the present invention is indicated by the following claims.

It should be appreciated that the present invention is not limited to the specific structures described above and illustrated in the drawings, and that various modifications and changes can be made without departing from the scope of the present invention. The scope of the present invention is limited only by the appended claims.

## Claims

1. A body temperature monitoring device (1), configured to work in cooperation with a mask (2) having a mask body (21) and a mask strap (22) provided with the mask body (21), comprising:
a strap connector (11) comprising a skin fitting portion (112) and a strap fitting portion (111) provided with at least one snap groove (1111) adapted to be fitted with the mask strap (22); and
a temperature measurement module (12) mounted to the strap connector (11) and arranged on the skin fitting portion (112).

2. The body temperature monitoring device (1) according to claim 1, further comprising a shaping member (13) fitted with at least a part of the strap connector (11).

3. The body temperature monitoring device (1) according to claim 2, wherein the shaping member (13) comprises a metal wire arranged within the strap connector (11).

4. The body temperature monitoring device (1) according to any one of claims 1 to 3, wherein:
the strap fitting portion (111) is provided with a plurality of snap grooves (1111);
the strap connector (11) comprises a central section (113); and
the plurality of snap grooves (1111) are symmetrically arranged with respect to the central section (113) in a length direction of the strap connector (11).

5. The body temperature monitoring device (1) according to any one of claims 1 to 4, wherein:
the temperature measurement module (12) comprises a heat conductive element (122) and a temperature sensor (121),
the heat conductive element (122) is arranged on a surface of the skin fitting portion (112); and
the temperature sensor (121) cooperates with the heat conductive element (122).

6. The body temperature monitoring device (1) according to claim 5, wherein:
the strap connector (11) comprises two skin fitting portions (112) located at both ends of the strap connector (11);
each of the two skin fitting portions (112) comprises an inner surface and an outer surface arranged oppositely; and
the heat conductive element (122) is arranged on one of the inner surface or the outer surface of at least one skin fitting portion (112) of the two skin fitting portions (112).

7. The body temperature monitoring device (1) according to claim 5 or 6, wherein the heat conductive element (122) comprises a metal plate.

8. The body temperature monitoring device (1) according to any one of claims 1 to 7, wherein the temperature measurement module (12) comprises an infrared transceiver.

9. The body temperature monitoring device (1) according to any one of claims 1 to 8, further comprising a wireless association module (123) electrically connected to the temperature measurement module (12) and associated with a mobile apparatus.

10. A mask assembly, comprising a mask (2) and the body temperature monitoring device (1) according to any one of claims 1 to 9, the mask (2) comprising a mask body (21) and a mask strap (22) provided to the mask body (21), and the body temperature monitoring device (1) cooperating with the mask strap (22).

11. An access control method, applied to the body temperature monitoring device (1) according to any one of claims 1 to 9, or the mask assembly according to claim 10, wherein the access control method comprises:
acquiring historical body temperature data for a period during which a user wears the body temperature monitoring device (1) or the mask assembly; and
sending the historical body temperature data to a mobile apparatus (3) and/or a cloud server (4).

12. An access control device (80), applied to the body temperature monitoring device (1) according to any one of claims 1 to 9 or the mask assembly according to claim 10, wherein the access control device (80) comprises:
a first acquiring unit (801) configured to acquire historical body temperature data for a period during which a user wears the body temperature monitoring device (1) or the mask assembly; and
a sending unit (802) configured to send the historical body temperature data to a mobile apparatus (3) and/or a cloud server (4).

13. An access control system, comprising:
the body temperature monitoring device (1) according to any one of claims 1 to 9 or a mask assembly according to claim 10, configured to acquire historical body temperature data during a period during which a user wears the body temperature monitoring device (1) or the mask assembly;
a cloud server (4) configured to store and manage at least a part of preset verification parameters, wherein the preset verification parameters comprise identity information and historical body temperature data corresponding to the identity information, and the part of the preset verification parameters comprises the historical body temperature data acquired by the body temperature monitoring device or the mask assembly and sent to the cloud server (4); and
an access control apparatus (5) coupled to the cloud server (4) and configured to release the access control when receiving an access control releasing signal.

14. The access control system according to claim 13, wherein the access control apparatus (5) is configured to send the access control releasing signal when the preset verification parameters meet the access control releasing condition.

15. The access control system according to claim 13, wherein the cloud server (4) is configured to send the access control releasing signal when the preset verification parameters meet the access control releasing condition.
